# EUROPEAN PATENT SPECIFICATION

(11) **EP 2 416 841 B1**
(45) Date of publication and mention of the grant of the patent: **23.09.2015**
(21) Application number: 09792072.2
(22) Date of filing: 28.08.2009
(51) Int. Cl.: A61N 1/375

(54) **ELECTRONICS PACKAGE FOR AN ACTIVE IMPLANTABLE MEDICAL DEVICE**
ELEKTRONIK-PAKET FÜR EINE WIRKSAME IMPLANTIERBARE MEDIZINISCHE VORRICHTUNG
BOÎTIER ÉLECTRONIQUE POUR UN DISPOSITIF MÉDICAL IMPLANTABLE ACTIF

(30) Priority: 08.04.2009 AU 2009901532
(43) Date of publication of application: 15.02.2012
(73) Proprietor: Saluda Medical Pty Limited, Eveleigh, NSW 2015 (AU)
(72) Inventor: PARKER, John, L., Alexandria New South Wales 1435 (AU)
(74) Representative: Legg, Cyrus James Grahame
(86) International application number: PCT/US2009/055405
(87) International publication number: WO 2010/117382

(56) References cited:
- WO-A-03/061517
- US-A- 4 837 049
- US-A1- 2002 028 991
- US-A1- 2005 246 002
- US-A1- 2006 173 259
- US-A1- 2007 123 766

## Description

### BACKGROUND

### Field of the Invention

The present invention relates generally to active implantable medical devices (AIMDs), and more particularly, to an electronics package for an AIMD.

### Related Art

Medical devices having one or more active implantable components, generally referred to herein as active implantable medical devices (AIMDs), have provided a wide range of therapeutic benefits to patients over recent decades. AIMDs often include an implantable, hermetically sealed electronics module, and a device that interfaces with a patient's tissue, sometimes referred to as a tissue interface. The tissue interface may include, for example, one or more instruments, apparatus, sensors or other functional components that are permanently or temporarily implanted in a patient. The tissue interface is used to, for example, diagnose, monitor, and/or treat a disease or injury, or to modify a patient's anatomy or physiological process.

In particular applications, an AIMD tissue interface includes one or more conductive electrical contacts, referred to as electrodes, which deliver electrical stimulation signals to, or receive signals from, a patient's tissue. The electrodes are typically disposed in a biocompatible electrically non-conductive member, and are electrically connected to the electronics module. The electrodes and the non-conductive member are collectively referred to herein as an electrode assembly.

WO 03/061217 A (California Institute of Technology), published 21 July 2003, discloses a neural prosthetic micro system that includes an electrode array coupled to an integrated circuit. An alignment plate is provided that includes holes corresponding to the positions of contact pads on the integrated circuit. The electrodes, being wire probes, are inserted in the holes and bonded to the contact pads.

US 2002/028991 A1 (Thompson David L), published 7 March 2002, discloses an epidermally mountable device including terminus structures to enable positive connection with conductive tissue. Nano spikes which are designed to penetrate the epidermis include a substrate comprising, preferably, a flexible metalized conductive plate attachable to an adhesive backing.

EP 0676717A (AT & T Corporation), published 11 October 1995, discloses improved methods for fabricating smart cards that have improved resistance to mechanical flexure, comprising a substrate, a frame surrounding the electronic components and a cover for hermetically sealing the electronic components.

US 2007/123766 A1 (Whenlen III, John), published 31 May 2007, discloses a microelectrode assembly that includes a two-side substrate and an array of microelectrodes. Each of the microelectrodes includes a nano-wire embedded within and extending through the substrate that remain fluid impermeable. The nano-wires can be adapted to be connected at one end to an electronic device and the other end to be disposed in a biological environment for biostimulating a target tissue and/or bio-sensing activities of the target tissue.

### SUMMARY The present invention is defined in the independent claims 1 and 5. Preferred embodiments are apparent from the claims dependent thereon.

In accordance with one aspect of the present invention, an electronics package for an active implantable medical device (AIMD) is provided.

In accordance with another aspect of the present invention, a method for manufacturing an electronics package for an active implantable medical device (AIMD) is provided.

### BRIEF DESCRIPTION OF THE DRAWINGS

Aspects and embodiments of the present invention are described herein with reference to the accompanying drawings, in which:
FIG. 1 is a perspective view of an exemplary active implantable medical device (AIMD), namely a neurostimulator, in accordance with embodiments of the present invention;
FIG. 2 is a functional block diagram of the deep brain stimulator illustrated in FIG. 1, in accordance with embodiments of the present invention;
FIG. 3A is a flowchart illustrating a method for manufacturing an electronics package in accordance with embodiments of the present invention;
FIG. 3B is a flowchart illustrating the operations performed during a manufacturing step of FIG. 3A;
FIG. 4A is a perspective view of a substrate having one or more active components thereon in accordance with embodiments of the present invention;
FIG. 4B is a side view of a substrate having one or more active components thereon in accordance with embodiments of the present invention;
FIG. 5A is an exploded view of an electronics package in accordance with embodiments of the present invention;
FIG. 5B is a cross-sectional view of the electronics package of FIG. 5A in accordance with embodiments of the present invention;
FIG. 6 is a cross-sectional view of two concurrently formed electronics packages in accordance with embodiments of the present invention;
FIG. 7 is an exploded view of an electronics package in accordance with embodiments of the present invention;
FIG. 8A is a cross-sectional view of an electronics package in accordance with embodiments of the present invention;
FIG. 8B is an exploded view of the electronics package of FIG. 8A in accordance with embodiments of the present invention;
FIG. 9A is a cross-sectional view of an electronics package in accordance with embodiments of the present invention;
FIG. 9B is a perspective view of the electronics package of FIG. 9A;
FIG. 9C is a cross-sectional view of an electronics package in accordance with embodiments of the present invention;
FIG. 10 is a perspective view of plurality of electrically connected electronics packages, in accordance with embodiments of the present invention;
FIG. 11 is a perspective view of plurality of electrically connected electronics packages, in accordance with embodiments of the present invention;
FIG. 12A is a perspective view of two electronics packages, in accordance with embodiments of the present invention;
FIG. 12B is a perspective view of a plurality of electrically connected electronics packages, in accordance with embodiments of the present invention;
FIG. 13A is a perspective view of two electronics packages, in accordance with embodiments of the present invention; and
FIG. 13B is a perspective view of a plurality of electrically connected electronics packages, in accordance with embodiments of the present invention.

### DETAILED DESCRIPTION

Aspects of the present invention are generally directed to an active implantable medical device (AIMD) comprising an implantable, hermetically sealed electronics package configured to interface with a patient's tissue. The electronics package comprises a biocompatible, electrically non-conductive and fluid impermeable planar substrate usable for semiconductor manufacturing that has one or more active components on the surface thereof. A biocompatible and fluid impermeable cover is bonded to the surface of the substrate to hermetically seal the active components between the substrate and the cover. The electronics package further comprises a conductive region formed on an exposed surface of the substrate or the cover that is electrically connected to at least one of the active components.

Embodiments of the present invention are described herein primarily in connection with one type of AIMD, a neurostimulator. Neurostimulators are a particular type of AIMD that deliver electrical stimulation, alone or in combination with other types of stimulation, to a patient's tissue. It should be appreciated that embodiments of the present invention may be implemented in any neurostimulator now know or later developed, such as brain stimulators (deep brain stimulators, cortical stimulators, *etc.),* cardiac pacemakers/defibrillators, functional electrical stimulators (FES), spinal cord stimulators (SCS), pain stimulators, *etc.* Embodiments of the present invention may also be implemented in AIMDs that are implanted for a relatively short period of time to address acute conditions, as well in AIMDs that are implanted for a relatively long period of time to address chronic conditions.

As used herein, neurostimulators in accordance with embodiments of the present are not limited to devices that deliver electrical stimulation signals to a patient. For instance, in certain embodiments, a neurostimulator may include one or more elements used to record or monitor the physiological response of a patient's tissue to, for example, a delivered therapy. In such embodiments, the neurostimulator receives a signal from the patient's tissue representing the tissue's physiological response. As described below, a neurostimulator may also include one or more other components, such as therapeutic agent delivery mechanisms, sensors, *etc.,* that interface with the patient's tissue.

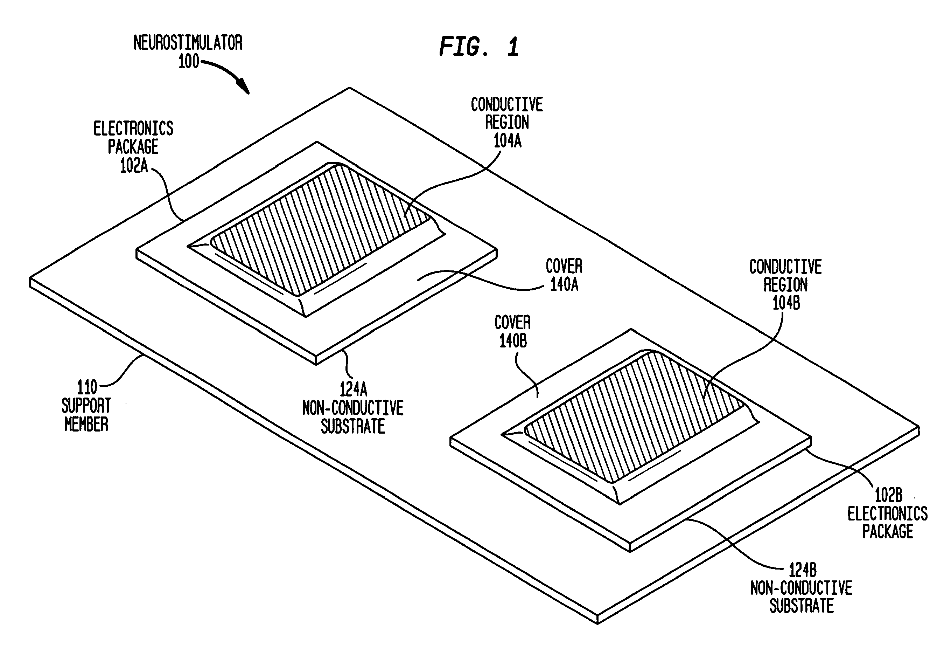

FIG. 1 is a perspective view of an active implantable medical device (AIMD), namely a neurostimulator 100, in accordance with embodiments of the present invention. Neurostimulator 100 comprises first and second electronics packages 102. As described in greater detail below, electronics packages 102 each comprise a biocompatible, non-conductive and fluid impermeable substrate 124. Substrates 124 are usable for semiconductor manufacturing and each have one or more active components (not shown) on the surface thereof. As used herein, an active component refers to any component that utilizes, or operates with, electrical signals. For example, an active component may comprise a stimulator unit, transceiver, power source, control module or other component used in an electronics module of an AIMD.

Electronics packages 102 each further comprise a biocompatible and fluid impermeable cover 140 that is bonded to the surface of substrate 124 to hermetically seal the active components between the substrate and the cover. Each package 102 further includes a conductive region 104 disposed on a surface of cover 140 that is electrically connected to at least one of the active components within the package. As described below, conductive regions 104 are used to deliver electrical stimulation signals to, or receive signals from, the tissue of a patient. In the illustrative embodiments of FIG. 1, electronics packages 102 are disposed on an elongate support member 110.

The embodiments of FIG. 1 illustrate the use of two electronics packages 102. It should be appreciated these embodiments are merely illustrative and that a greater or lesser number of electronics packages may be provided. As noted, FIG. 1 also illustrates the electronics packages 102 disposed on a support member 110. As described below, the use of such a support member is unnecessary in some embodiments of the present invention.

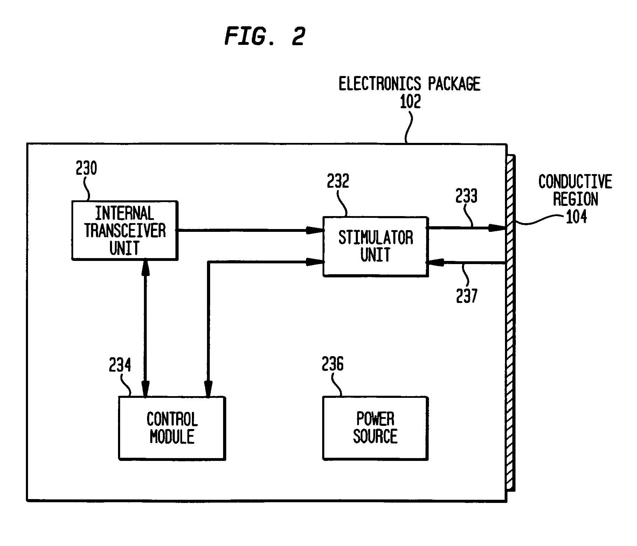

FIG. 2 is functional block diagram of embodiments of electronics package 102 of neurostimulator 100. In these embodiments, electronics package 102 includes one or more functional components which facilitate the delivery of stimulation signals to, or reception of signals from, a patient's tissue. More specifically, electronics package 102 comprises an internal transceiver unit 230 that may form bi-directional transcutaneous communication links with transceiver units (not shown) in other implanted or external devices. For instance, in certain embodiments, electronics package 102 transcutaneously communicates with other electronics packages. Transceiver unit 230 includes a collection of one or more components configured to receive and/or transfer power and/or data. Transceiver unit 230 may comprise, for example, a coil for a magnetic inductive arrangement, a capacitive plate, or any other suitable arrangement.

In the specific embodiment of FIG. 2, electronics package 102 further includes a stimulator unit 232 that generates electrical stimulation signals 233. Electrical stimulation signals 233 are delivered to a patient's tissue via conductive region 104 on the exterior surface of electronics package 102. Stimulator unit 232 may generate electrical stimulation signals 233 based on, for example, data received from another device, signals received from a control module 234, in a pre-determined or pre-programmed pattern, *etc.*

As noted above, in certain embodiments of the present invention, conductive region 104 is configured to record or monitor the physiological response of a patient's tissue. As shown, signals 237 representing the recorded response may be provided to stimulator unit 232 for forwarding to control module 234, or to another device via, for example, a transcutaneous communication link.

In the embodiments of FIG. 2, electronics package 102 is a totally implantable medical device that is capable of operating, at least for a period of time, without the need for an external device. Therefore, electronics package 102 further comprises a power source 236. Power source 236 may comprise, for example, a rechargeable battery that stores power received from an external device, or from other implanted devices. During operation of electronics package 102, the power stored by power source 236 is distributed to the various other components of electronics package 102 as needed. For ease of illustration, electrical connections between power source 236 and the other components of electronics package 102 have been omitted.

It should be appreciated that the embodiments of FIG. 2 are merely illustrative and that other arrangements for electronics packages 102 are within the scope of the present invention. For example, an electronics package in accordance with embodiments of the present invention may include other components therein that are not shown in FIG. 2 that are suitable to facilitate the operation of other instruments, apparatus, sensors, processors, controllers or other functional components used to, for example, diagnose, monitor, and/or treat a disease or injury, or to modify the patient's anatomy or physiological process.

As previously noted, embodiments of the present invention are directed to an AIMD comprising an implantable, hermetically sealed electronics package that is configured to interface with a patient's tissue. The electronics package comprises a biocompatible, non-conductive and fluid impermeable substrate usable for semiconductor manufacturing that has one or more active components on the surface thereof. A biocompatible and fluid impermeable cover is bonded to the surface of the substrate to hermetically seal the active components between the substrate and the cover. The electronics package further comprises a conductive region formed on an exposed surface of the substrate or the cover that is electrically connected to at least one of the active components.

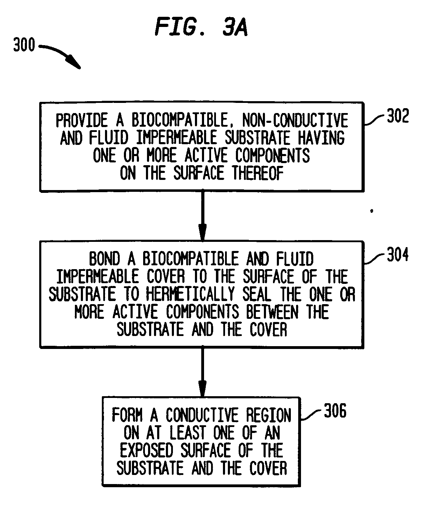

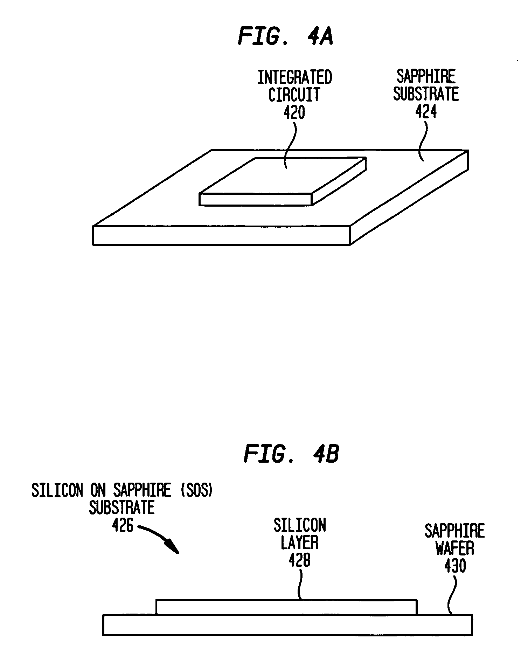

FIG. 3A is a flowchart illustrating a method 300 of manufacturing an electronics package in accordance with embodiments of the present invention. Method 300 begins at block 302 where a biocompatible, non-conductive and fluid impermeable substrate having one or more active components on the surface thereof is provided. Substrates of the present invention are suitable for use in semiconductor device fabrication (i.e. in the production of electronic components and integrated circuits). Details of exemplary substrates are provided with reference to FIGS. 4A and 4B.

The steps of block 302 may further involve the bonding of an integrated circuit (IC) to the substrate or the formation of active electronic circuits in the substrate surface, as described below with reference to FIGS. 4A and 4B. This step may also include preparation of the substrate for bonding to the IC, for formation of the active circuits therein, and/or planarization of the substrate surface.

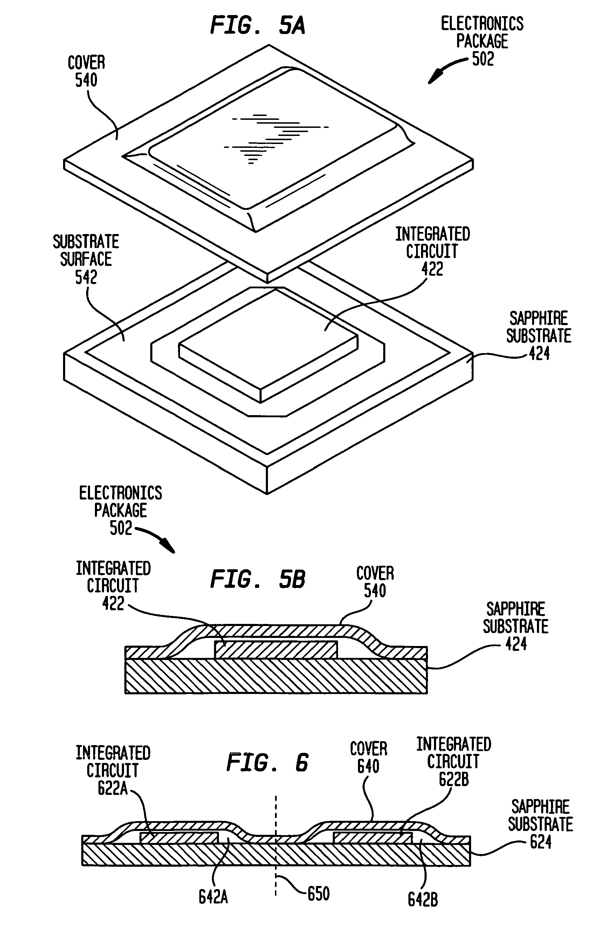

At block 304, a biocompatible and fluid impermeable cover is bonded to the surface of the substrate to hermetically seal the one or more active components between the substrate and the cover. As described below with reference to FIGS. 5A and 5B, the cover may comprise the same or different material as the utilized substrate. Exemplary methods for bonding the cover to the substrate are also described below with reference to FIGS. 5A and 5B.

At block 306, a conductive region is formed on an exposed surface of the substrate, or on an exposed surface of the cover. It should be appreciated that this conductive region may be formed prior to, or following the bonding step of block 304.

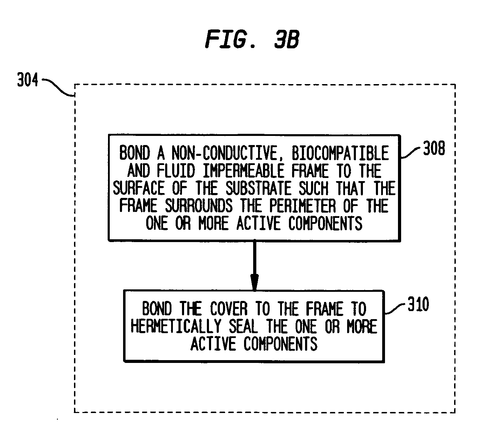

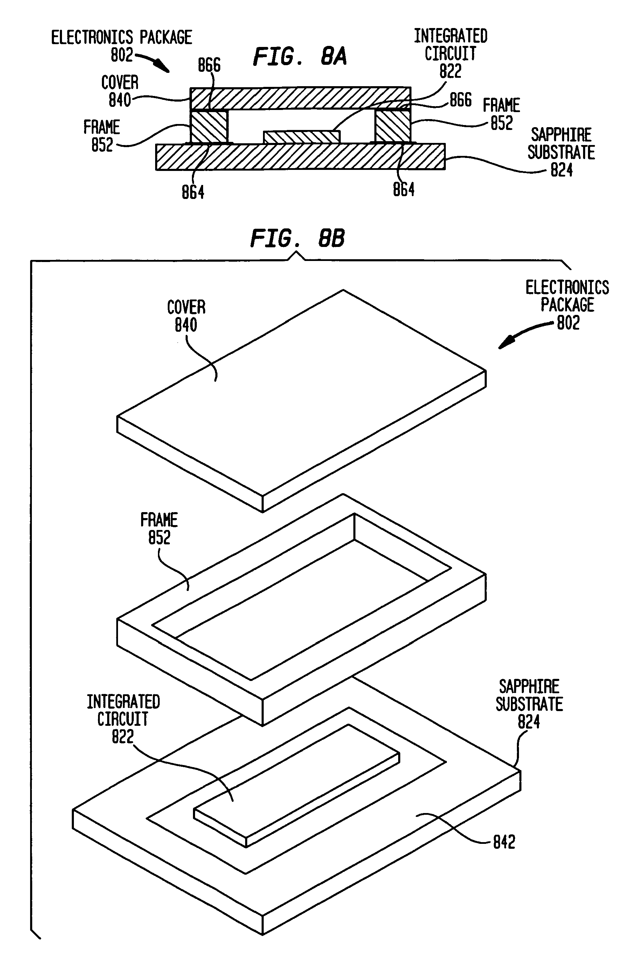

In certain embodiments of the present invention, the cover is bonded directly to the substrate at block 304. FIG. 3B is a flowchart illustrating alternative embodiments of the present invention. In the embodiments of FIG. 3B, a non-conductive, biocompatible and fluid impermeable frame is bonded to the surface of the substrate at block 308. The frame and substrate are bonded such that the frame surrounds the perimeter of the active components on the substrate. At block 310, the cover is bonded to the frame to hermetically seal the active components between the cover, frame and substrate. Exemplary such embodiments are described below with reference to FIGS. 8A- 9B.

As noted, FIGS. 4A and 4B illustrate exemplary biocompatible, non-conductive and fluid impermeable substrates that may be utilized in embodiments of the present invention. Such substrates are suitable for use in semiconductor device fabrication (i.e. in the production of electronic components and integrated circuits), and do not degrade when exposed to an implanted environment. In certain embodiments, the substrates are configured to be metallically bonded to a cover at a temperature below 400 degrees Celsius. Depending on the desired application, suitable substrates may include, for example, sapphire, silicon or ceramic.

Also as noted, a substrate in accordance with embodiments of the present invention has one or more active components thereon. In the embodiments of FIG. 4A, the active components are elements of an Integrated Circuit (IC) 420. In these embodiments, a sapphire substrate 424 is manufactured in such a way as to allow IC 420 to be attached thereto via, for example, wafer bonding. FIG. 4B illustrates alternative embodiments of the present invention in which a substrate 426 is formed using silicon-on-sapphire (SOS) processing. SOS processing utilizes a layer of silicon 428 on a sapphire wafer 432. In such embodiments, the active components comprise circuit elements (not shown) that are formed directly in silicon layer 428. SOS processing is known in the art and will not be described further herein.

FIG. 5A is an exploded view of an electronics package 502 in accordance with embodiments of the present invention, while FIG. 5B is a cross-sectional view of electronics package 502. For ease of illustration, the embodiments of FIGS. 5A and 5B will be discussed with reference to substrate 424 having an IC 422 thereon, as described above with reference to FIG. 4A.

To form electronics package 502 shown in FIG. 5B, a biocompatible and fluid impermeable cover 540 is bonded to a surface 542 of sapphire substrate 424. Cover 540 may be the same or different material as substrate 424. For example, cover 540 may be formed from sapphire, silicon, ceramic, platinum, titanium or other biocompatible material. It should be appreciated that the substrates of FIGS. 4A and 4B may each be paired with any suitable cover.

As noted, cover 540 is bonded to substrate such that IC 422 is hermetically sealed between the cover and the substrate. In certain embodiments, surface 542 is planarized and/or polished to prepare the surface for bonding. The bonding surface (not shown) of cover 540 may also be prepared in a similar manner as surface 542.

It should be appreciated that a number of bonding methods may be used to bond cover 540 to surface 542. One exemplary method is silicon fusion bonding. Silicon fusion bonding is a high temperature process where silicon wafers are bonded at 1000C. It will be apparent to those skilled in the art that the appropriate materials for substrate and cover may be selected to permit use of this bonding method. For instance, a platinum cover may be an appropriate cover material due to the fact that platinum boils at higher temperatures than is required for the fusion bonding process.

Thin metal film bonding is a still other possible bonding method. In this method, thin metal films are deposited on the surfaces of cover 540 and substrate 424. The surfaces are brought together during, or immediately following the deposition, thereby allowing the thin metal films to diffuse and form a bond.

Another method for bonding cover 540 to surface 542 is anodic bonding. In these embodiments, the bonding occurs between a sodium rich glass substrate and a polysilicon film. The bond is formed at temperatures and voltage (typically 1000 Volts) which mobilize the ions in the glass. The applied potential causes the sodium to deplete from the bonding interface and an electrostatic bond is formed.

Reactive metal film bonding is another method that may be implemented in certain embodiments. In this method, a reactive multilayer foil is used as a heat source to bond a sapphire substrate and metal cover. The sapphire substrate and metal cover each have a solder (or braze) layer deposited on the mating surface. The reactive multi-layer foil is placed between the two solder layers of the sapphire substrate and metal cover which are brought together at room temperature under vacuum. The heat generated by the reaction of the foil fuses the solder/braze material and forms a hermetic bond. The reactive foil is aluminium, nickel or a similar metal/alloy of approximately 40 to 100 micrometers.

Laser brazing is another possible bonding technique. In this method the sapphire substrate and a metal cover are bonded by the use of a braze alloy or glass deposited on the sapphire substrate. The cover and substrate are brought together, and a CO₂ laser is used to heat the joint to a level above the austenitizing temperature to form a hermetic bond. A suitable braze material may be, for example, TiCuNi.

Solder bonding is another bonding method that may be utilized. Solder bonding occurs at low temperature and a variety of materials are available to perform this type of bond. For example, there are gold based solders which may be sufficiently stable and biocompatible for a medical application.

A still other bonding method is silicide direct bonding. This method is used to bond a silicon PtSi coated wafer, and one of either a PtSi coated or uncoated silicon wafer. This type of bonding occurs when the PtSi surface is rendered hydrophilic by a hot aqua regia selective etching and cleaning process. The PtSi provides bondable, relatively low resistance paths which provide electrical interconnections between circuit elements on the bonded pair of wafers.

Another bonding process is known as eutectic alloy bonding. This type of bonding occurs when a metal forms a eutectic alloy at the interface of the bond. A form of this bonding has been demonstrated with Platinum and Titanium as the metals.

As noted, an electronics package 502 in accordance with embodiments of the present invention may be configured to communicate with other implanted devices. In certain such embodiments, cover 540 comprises an optically transparent material. In these embodiments, data may be transmitted to the active components within electronics package 502. A photodiode mounted in package 502 detects the transmitted data. A sapphire cover may be used in such embodiments due to the fact that sapphire is optically transparent in the infrared (IR) region. Alternative embodiments may use a glass cover.

FIG. 6 is a cross-sectional view of an alternative embodiment of the present invention in which multiple electronics packages are formed using a single bonding step. In these embodiments, a sapphire substrate 624 is prepared. Sapphire substrate 624 has two physically spaced ICs 622 mounted on the surface thereof. A cover 640 having multiple cavities 642 is bonded to substrate 624 such that each IC 622 is hermetically sealed within one of the cavities 642. Substrate 624 and cover 640 may then be cut along line 650 to form two independent electronics packages.

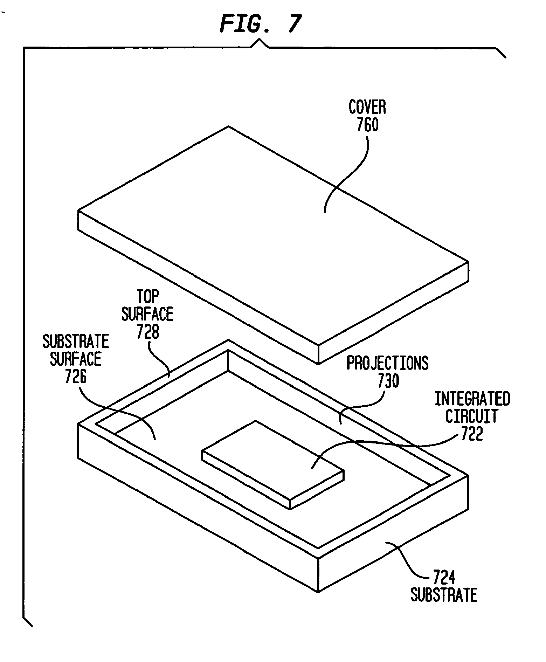

FIG. 7 illustrates an alternative embodiment of the present invention in which a substrate 724 is bonded to a cover 760. In the embodiments of FIG. 7, substrate 724 comprises a planar surface 726 having an integrated circuit 722 mounted thereon. However, in the embodiments of FIG. 7, substrate 724 further comprises four contiguous projections 730 extending from surface 726. Projections 730 extend about the perimeter of IC 722. Cover 760 is bonded to the top surface 728 of projections 730 to hermetically seal IC 722 between cover 760, projections 730 and surface 726.

FIGS. 8A and 8B are cross-sectional and exploded views, respectively, of an electronics package 802 in accordance with embodiments of the present invention. In these embodiments, electronics package 802 comprises a sapphire substrate 824 that is substantially similar to the substrate described above with reference to FIG. 4A. Bonded to the surface of substrate 824 is an integrated circuit (IC) 822.

As shown, electronics package 802 further comprises a biocompatible, electrically non-conductive and fluid impermeable frame 852 bonded to the surface of substrate 824. Frame 852 surrounds the perimeter of IC 822. A cover 840, which is substantially similar to the cover described above with reference to FIGS. 5A and 5B, is bonded to frame 852 to hermetically seal IC 822 between cover 840, frame 852 and substrate 824.

It should be appreciated that any of the bonding methods described above with reference to FIGS. 5A and 5B may be used to bond frame 852 to substrate 824. In certain embodiments of the present invention, a conductive material may be used to form the bond between frame 852 and substrate 824. The conductive bonding material may be configured to extend beyond the bonding area (i.e. the area directly between the lower surface of frame 852 and substrate 824). This creates a conductive pathway 864 that extends from a section of the surface of substrate 824 within the area enclosed by frame 852, to a section of the surface of the substrate outside the frame. Conductive pathway 864 functions as feed through which may be used to connect IC 822 to components external to electronics package 802. A similar feed through 866 may be fabricated using a metallic bond between non-conductive cover 840 and frame 852.

It would be appreciated that various methods may be implemented to connected IC 822 to conductive pathways 864, 866. For example, wires (not shown) may be connected between IC 822 and conductive pathways 864, 866..

In further embodiments of the present invention, frame 852 may be bonded to substrate 824 to provide a hermetic feed through the frame. More specifically, in such embodiment an aperture may be formed through frame 852. A first opening of the aperture would be electrical contact with a modified conductive layer 864 that extends within the sealed cavity, but which does not extend outside the perimeter of the frame. The other surface of the aperture is on the exterior surface of the device, and the aperture may be converted to a conductive via. Thus, a conductive pathway from the via through layer 864 extends to the interior of the cavity. In further embodiments, cover 840 or substrate 824 may formed and bonded to frame 852.

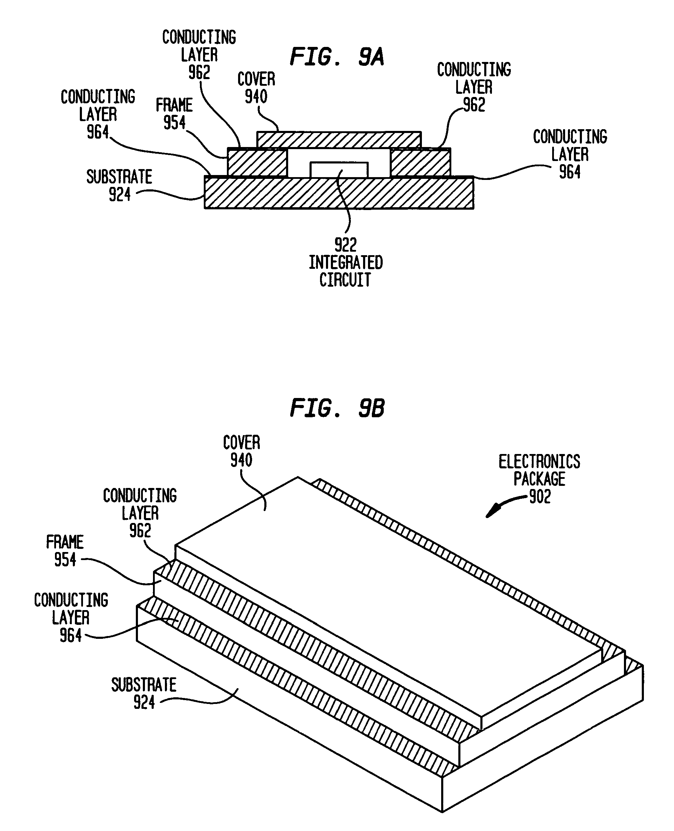

FIGS. 9A and 9B are cross-sectional and perspective views of an electronics package 902 in accordance with embodiments of the present invention. In these embodiments, electronics package 902 comprises a sapphire substrate 924 that is substantially similar to the substrate described above with reference to FIG. 4A. Bonded to the surface of substrate 924 is an integrated circuit (IC) 922.

As shown, electronics package 902 further comprises a biocompatible, electrically non-conductive and fluid impermeable frame 954 bonded to the surface of substrate 924. Frame 954 surrounds the perimeter of IC 922. A cover 940, which is substantially similar to the cover described above with reference to FIGS. 5A and 5B, is bonded to frame 954 to hermetically seal IC 922 between cover 940, frame 954 and substrate 924. It should be appreciated that any of the bonding methods described above with reference to FIGS. 5A and 5B may be used to bond frame 852 to substrate 824.

In the embodiments of FIGS. 9A and 9B, a section of substrate 924 has a conducting layer 964 thereon. Conducting layer 964 is disposed on substrate 924 such that when frame 954 is bonded thereto, a first portion of layer 964 will be within the hermetically sealed cavity of electronics package 902, while a second portion of layer 964 will be on an exposed surface of electronics package 902. Frame 954 hermetically seals the first and second portions of layer 964 from one another, thereby forming a hermetically sealed conductive pathway between IC 922 and the exposed surface of electronics package 902. The exposed portion of layer 964 may be used to connect electronics package 902 to one or more other components, to deliver electrical stimulation signals to a patient's tissue, receive signals from a patient's tissue, *etc.*

In the embodiments of FIGS. 9A and 9B, the surface of frame 954 that is to be bonded to cover 940 has a conducting layer 962 disposed thereon. Cover 940 has dimensions selected so that when the cover and frame 954 are bonded, a first portion of layer 962 will be within the hermetically sealed cavity of electronics package 902, while a second portion of layer 962 will be on an exposed surface of electronics package 902. Cover 940 hermetically seals the first ands second portions of layer 962 from one another, thereby forming a hermetically sealed conductive pathway between IC 922 and the exposed surface of electronics package 902. The exposed portion of layer 962 may be used to connect electronics package 902 to one or more other components, to deliver electrical stimulation signals, receive signals from a patient's tissue, *etc.* In alternative embodiments, cover 940 or substrate 924 may formed and bonded to frame 954.

It would be appreciated that various methods may be implemented to connected IC 922 to conducting layers 962, 964. For example, wires (not shown) may be connected between IC 922 and conductive pathways 962, 964.

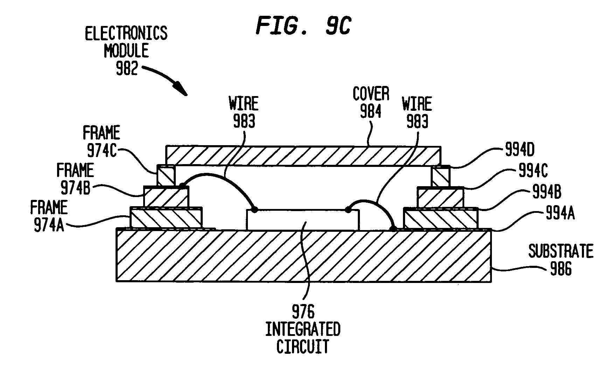

FIGS. 8A-9B illustrate embodiments in which a single frame is used between a cover and a substrate. However, according to the invention, a plurality of frames are be used to provide more than two conductive pathways between the hermetically sealed cavity and the exposed surface of an electronics package. One exemplary such embodiment is illustrated in FIG. 9C.

In the embodiments of FIG. 9C, electronics package 982 comprises a sapphire substrate 986 that is substantially similar to the substrate described above with reference to FIG. 4A. Bonded to the surface of substrate 986 is an integrated circuit (IC) 976. Electronics package 982 comprises a series of stacked biocompatible, electrically non-conductive and fluid impermeable frames 974 bonded to the surface of substrate 986. Frames 974 surround the perimeter of IC 976. A cover 984, which is substantially similar to the cover described above with reference to FIGS. 5A and 5B, is bonded to frame 974C to hermetically seal IC 976 between cover 984, frames 974, and substrate 986. A conductive pathway 994 is provided between each of substrate 986, frames 974 and cover 984, and are connected to IC 976 via, for example, wires 983. For ease of illustration, one two wires 983 are shown. Conductive pathways 994 may be formed in accordance with the embodiments described above with reference to FIGS. 8A and 8B, or FIGS. 9A and 9B.

In further embodiments, substrates 994, cover 940 and/or substrate 986 may formed and bonded to one another to form a hermetic feed through from the external environment to integrated circuit 976. It would be appreciated that the use of multiple frames 994 provides the ability to form multiple electrically separate feed throughs.

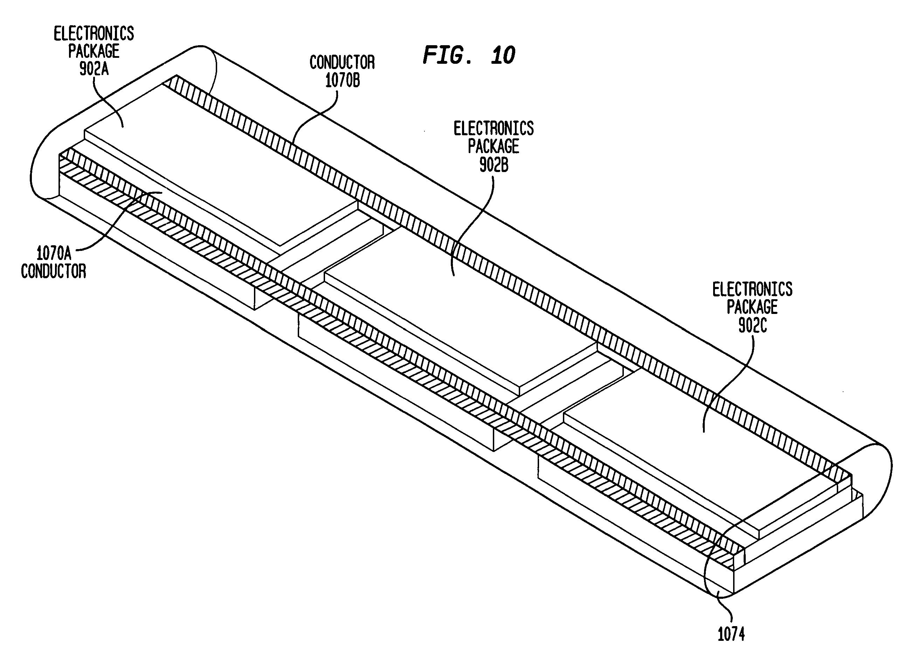

FIG. 10 illustrates embodiments of the present invention in which three electronics packages 902, as described above with reference to FIGS. 9A and 9B, are aligned in a linear arrangement. In these embodiments, conductors 1070 extend between exposed portions of conducting layers 962 (FIG. 9B) to electrically connect electronic packages 902 to one another. Conductors 1070 may be used to carry power and/or data between packages 902.

In embodiments of the present invention, packages 902 may be attached to, or at least partially embedded in a support structure. FIG. 10 illustrates specific embodiments in which packages 902 are imbedded in a flexible support structure 1074. A suitable support structure 1074 may comprise, for example, silicon, rubber, *etc.* As noted above, electronics packages 902 have one more conductive regions disposed thereon that are configured to interface with a patient's tissue. It should be appreciated that in embodiments of the present invention these conductive regions are not embedded in support structure 1074.

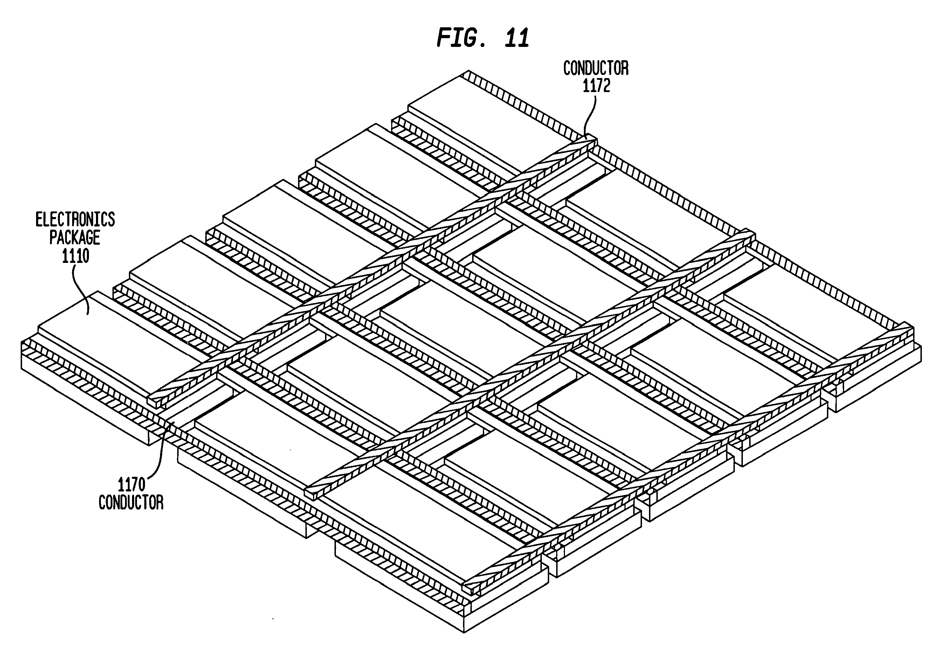

As noted, FIG. 10 illustrates embodiments of the present invention in which three packages 902 are arranged in a linear array. It should be appreciated that any number of packages may be arranged in a variety of different patterns. FIG. 11 illustrates one such alternative pattern in which five parallel linear rows of packages 902 are shown. Each package within a row is electrically connected to one another, as described above with reference to FIG. 10, by conductors 1170. Furthermore, the parallel rows are electrically connected to one another by additional conductors 1172. In one specific embodiment of the present invention, one of the packages includes an addressable switch which allows power and/or data to be sent to a desired package.

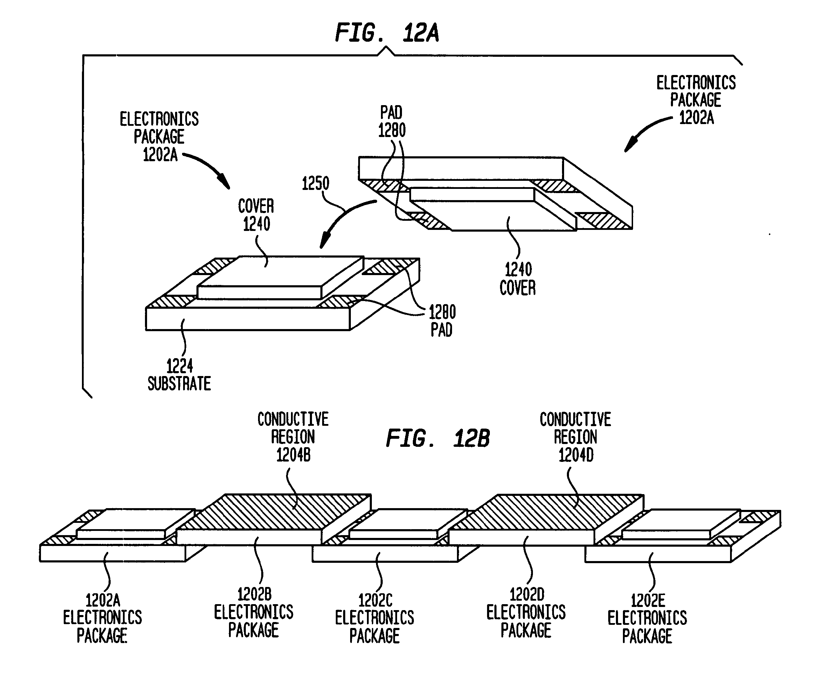

FIG. 12A is a perspective view of two electronics packages 1202A, 1202B in accordance with embodiments of the present invention. Similar to the embodiments described above, electronics packages 1202 each comprise a substrate 1224 having one or more active components thereon. A cover 1240 hermetically seals the active components between the cover and substrate 1224. Each electronics package 1202 further comprises one or more conductive pads 1280 that are electrically connected to at least one of the active components. For ease of illustration, only the relevant components of electronics package 1202B have been labeled in FIG. 12A.

In the embodiments of FIG. 12A, electronics package 1202A is shown having cover 1240 facing a first direction, while electronics package 1202B is shown having cover 1240 facing a second direction. As indicated by arrow 1250, opposing bond pads 1280 of electronics packages 1202 are brought together to electrically connect the electronics packages. FIG. 12B illustrates specific embodiments in which a plurality of electronics packages 1202 are connected using the process described with reference to FIG. 12A.

As noted above, electronics packages in accordance with embodiments of the present invention include a conductive region disposed on the exterior surface of the substrate or cover. In the arrangement of FIG. 12B, electronics packages 1202B ands 1202D each have a conductive region 1204 disposed on the surface of the substrate 1224. Conductive regions 1204 comprise a coating of a thin metallic film. This film is electrically connected to the active components inside the electronics package, thereby facilitating the delivery of electrical stimulation signals to, or reception of signals from, the tissue of a patient.

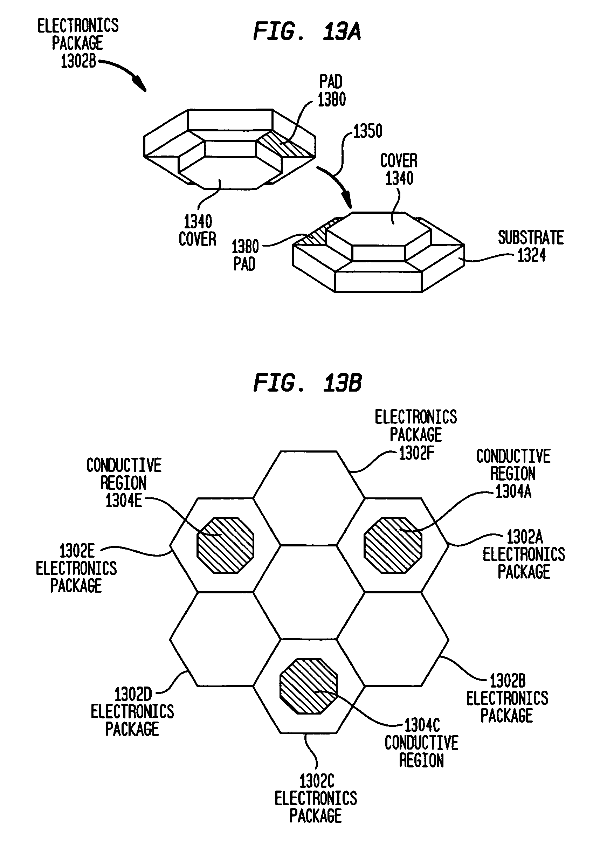

Embodiments of the present invention have been described primarily herein with reference to electronics packages manufactured from components having generally square or rectangular shapes. It should be appreciated that covers, substrates and frames in accordance with embodiments of the present invention may have a variety of shapes. FIGS. 13A and 13B illustrate embodiments of the present invention in which utilized substrates and covers have a generally hexagonal shape.

FIG. 13A is a perspective view of two electronics packages 1302A, 1302B in accordance with embodiments of the present invention. Similar to the embodiments described above, electronics packages 1302 each comprise a substrate 1324 having one or more active components thereon. A cover 1340 hermetically seals the active components between the cover and substrate 1324. Each electronics package 1302 further comprises one or more conductive pads 1380 that are electrically connected to at least one of the active components. For ease of illustration, only the relevant components of electronics package 1302B have been labeled in FIG. 13A.

In the embodiments of FIG. 13A, electronics package 1302A is shown having cover 1340 facing a first direction, while electronics package 1302B is shown having cover 1340 facing a second direction. As indicated by arrow 1350, opposing bond pads 1380 of electronics packages 1302 are brought together to electrically connect the electronics packages. FIG. 13B illustrates specific embodiments in which a plurality of electronics packages 1302 are connected using the process described with reference to FIG. 13A.

As noted above, electronics packages in accordance with embodiments of the present invention include a conductive region disposed on the exterior surface of the substrate or cover. In the arrangement of FIG. 13B, electronics packages 1302A, 1302C and 1302E each have a conductive region 1304 disposed on the surface of the substrate 1324. In these embodiments, conductive regions 1304 comprise a coating of a thin metallic film. This film is electrically connected to the active components inside the electronics package, thereby facilitating the delivery of electrical stimulation signals to, or reception of signals from, the tissue of a patient.

As is known in the art, hermetically sealed implantable medical devices are tested prior to use to verify the integrity of the hermetic seal. This verification ensures that the hermetic package is safe for long term use. A helium leak test is typically used to test the hermetic seal of conventional implantable devices. However, electronics packages in accordance with embodiments of the present invention may be too small for such helium leak testing due to the fact that an insufficient volume of required gas is present in the package. As such, embodiments of the present invention use an optical interferometer leak test to verify the hermetic integrity of a fabricated electronics package. Optical interferometer leak testing relies on an optical measurement of the deflection of the cover over a period of time while under external applied pressure or temperature. The optical measurement is made using an optical interferometer. Optical interferometer leak testing is known in the art and will not be described further herein.

## Claims

1. An electronics package (102) for an active implantable medical device (AIMD), comprising:
a biocompatible, electrically non-conductive and fluid impermeable planar substrate (986) usable for semiconductor manufacturing;
one or more active components (976);
a first biocompatible, electrically non-conductive and fluid impermeable frame (974A) attached to a surface of the substrate (986) such that the frame (974A) surrounds the perimeter of the one or more active components (976);
one or more second biocompatible, electrically non-conductive and fluid impermeable frames (974) attached to a surface of the first frame (974A) or to other second frame (974B);
a biocompatible and fluid impermeable cover (984) bonded to a surface of a second frame (974C) to hermetically seal the one or more active components (976) between the substrate (986) and the cover (984);
a conductive region (104) formed on at least one of an exposed surface of the substrate (986) and the cover (984) electrically connected to at least one of the one or more hermetically sealed active components (976); and
at least one conductive pathway (994) is provided for each frame (974) to electrically connect at least one of the one or more hermetically sealed active components (976) with at least one of the conductive region (104) and a component external to the electronics package.

2. The electronics package of claim 1, wherein the conductive region (104) is configured to interface with tissue of a recipient of the AIMD, and wherein at least one of the one or more active components comprises a stimulator unit configured to generate electrical stimulation signals delivered to the tissue by the conductive region (104).

3. The electronics package of claim 1, wherein the conductive region (104) is configured to interface with tissue of a recipient of the AIMD, and wherein the conductive region is configured to sense a nerve impulse generated by the tissue, and transmit an electrical signal representing the nerve impulse to at least one active component electrically connected thereto.

4. The electronics package of claim 1, further comprising a second conductive region configured to be electrically connected to a bond pad (1280) of a second electronics package (1202A).

5. A method for manufacturing an electronics package (102) for an active implantable medical device (AIMD), comprising:
providing (302) a biocompatible, electrically non-conductive and fluid impermeable planar substrate (986) having one or more active components (976);
bonding (308) a first biocompatible, electrically non-conductive and fluid impermeable frame (974A) to the surface of the substrate (986) such that the frame (974A) surrounds the perimeter of the one or more active components (976);
bonding one or more second biocompatible, electrically non-conductive and fluid impermeable frames (974) to a surface of the first frame (974A) or other second frame (974B);
bonding (310) a biocompatible and fluid impermeable cover (984) to a surface of a second frame (974C) to hermetically seal the one or more active components (976) between the substrate (986) and the cover (984);
forming (310) a conductive region (104) on at least one of an exposed surface of the substrate (986) and the cover (984), the conductive region electrically connected to at least one of the one or more hermetically sealed active components (976); and
forming at least one conductive pathway (994) for each frame (974) to electrically connect at least one of the one or more hermetically sealed active components (976) with at least one of the conductive region (104) and a component external to the electronics package.

6. The method of claim 5, wherein providing the substrate comprises:
bonding an integrated circuit comprising the one or more active components to the surface of a substrate.

7. The method of claim 5, wherein providing the substrate comprises: fabricating the one or more active components directly in the surface of the substrate.

8. The method of claim 5, further comprising: electrically connecting the conductive region to a second electronics package.

9. The method of claim 5, wherein forming the conductive region (104) comprises:
depositing a metallic thin film on the at least one of an exposed surface of the substrate and the cover.

10. The method of claim 5, wherein substrate comprises a plurality of regions each comprising one or more active components, and wherein the method further comprises:
bonding a cover (640) having a plurality of components to the substrate (624) such that each of the plurality of regions of the substrate (624) are hermetically sealed from one another.

11. The method of claim 10, further comprising:
separating the plurality of regions from one another such that the substrate is divided into a plurality of hermetically sealed and physically separate modules each having a conductive region on an exterior surface thereof.

12. The method of claim 5, further comprising: testing the integrity of the hermetic seal between the substrate (124) and the cover (140) by performing an optical interferometer leak test to test the integrity of the hermetic seal.

13. The electronics package of claim 1, wherein one or more active components comprise an integrated circuit bonded to at least one of the substrate and the cover.

14. The electronics package of claim 1, wherein one or more active components are formed directly in the surface of at least one of the substrate and the cover.

15. An electronic package of claim 1, wherein a conductive pathway (994) is provided for a frame by being provided between that frame and one of the substrate (986), other second frame (994) or cover (984).

16. An electronic package of claim 1, wherein a conductive pathway is provided for a frame (994) as an aperture through that frame.

## Patentansprüche

1. Elektronikgehäuse (102) für eine aktive implantierbare medizinische Vorrichtung (AIMD), das umfasst:
ein biokompatibles, nicht elektrisch leitendes und nicht fluiddurchlässiges ebenes Substrat (986), das zur Halbleiterfertigung verwendet werden kann;
eine oder mehrere aktive Komponenten (976);
einen ersten biokompatiblen, nicht elektrisch leitenden und nicht fluiddurchlässigen Rahmen (974A), der an einer Oberfläche des Substrats (986) in der Weise befestigt ist, dass der Rahmen (974A) den Umfang der einen oder der mehreren aktiven Komponenten (976) umgibt;
einen oder mehrere zweite biokompatible, nicht elektrisch leitende und nicht fluiddurchlässige Rahmen (974), die an einer Oberfläche des ersten Rahmens (974A) oder an einem anderen zweiten Rahmen (974B) befestigt sind;
eine biokompatible und nicht fluiddurchlässige Abdeckung (984), die mit einer Oberfläche eines zweiten Rahmens (974C) verbunden ist, um die eine oder die mehreren aktiven Komponenten (976) zwischen dem Substrat (986) und der Abdeckung (984) hermetisch abzudichten;
einen leitenden Bereich (104), der an einer freiliegenden Oberfläche des Substrats (986) und/oder der Abdeckung (984) gebildet ist und mit wenigstens einer der einen oder der mehreren hermetisch abgedichteten Komponenten (976) elektrisch verbunden ist; und
wobei für jeden Rahmen (974) wenigstens ein leitender Pfad (994) vorgesehen ist, um wenigstens eine der einen oder der mehreren hermetisch abgedichteten aktiven Komponenten (976) mit dem leitenden Bereich (104) und/oder einer Komponente außerhalb des Elektronikgehäuses elektrisch zu verbinden.

2. Elektronikgehäuse nach Anspruch 1, wobei der leitende Bereich (104) konfiguriert ist, eine Grenzfläche mit Gewebe eines Empfängers der AIMD zu bilden, und wobei wenigstens eine der einen oder der mehreren aktiven Komponenten eine Stimulatoreinheit umfasst, die konfiguriert ist, elektrische Stimulationssignale zu erzeugen, die durch den leitenden Bereich (104) an das Gewebe verabreicht werden.

3. Elektronikgehäuse nach Anspruch 1, wobei der leitende Bereich (104) konfiguriert ist, eine Grenzfläche mit Gewebe eines Empfängers der AIMD zu bilden, und wobei der leitende Bereich konfiguriert ist, einen durch das Gewebe erzeugten Nervenimpuls zu erfassen und ein elektrisches Signal, das den Nervenimpuls darstellt, an wenigstens eine damit elektrisch verbundene aktive Komponente zu senden.

4. Elektronikgehäuse nach Anspruch 1, das ferner eine zweiten leitenden Bereich umfasst, der konfiguriert ist, mit einer Bond-Anschlussfläche (1280) eines zweiten Elektronikgehäuses elektrisch verbunden zu werden.

5. Verfahren zum Herstellen eines Elektronikgehäuses (102) für eine aktive implantierbare medizinische Vorrichtung (AIMD), das umfasst:
Bereitstellen (302) eines biokompatiblen, nicht elektrisch leitenden und nicht fluiddurchlässigen ebenen Substrats (986), das eine oder mehrere aktive Komponenten (976) besitzt;
Verbinden (308) eines ersten biokompatiblen, nicht elektrisch leitenden und nicht fluiddurchlässigen Rahmens (974A) mit der Oberfläche des Substrats (986) in der Weise, dass der Rahmen (974A) den Umfang der einen oder der mehreren aktiven Komponenten (976) umgibt;
Verbinden eines oder mehrerer zweiter biokompatibler, nicht elektrisch leitender und nicht fluiddurchlässiger Rahmen (974) mit einer Oberfläche des ersten Rahmens (974A) oder eines anderen zweiten Rahmens (974B);
Verbinden (310) einer biokompatiblen und nicht fluiddurchlässigen Abdeckung (984) mit einer Oberfläche eines zweiten Rahmens (974C), um die eine oder die mehreren aktiven Komponenten (976) zwischen dem Substrat (986) und der Abdeckung (984) hermetisch abzudichten;
Bilden (310) eines leitenden Bereichs (104) auf einer freiliegenden Oberfläche des Substrats (986) und/oder der Abdeckung (984), wobei der leitende Bereich mit wenigstens einer der einen oder der mehreren hermetisch abgedichteten aktiven Komponenten (976) elektrisch verbunden ist; und
Bilden wenigstens eines leitenden Pfades (994) für jeden Rahmen (974), um wenigstens eine der einen der mehreren hermetisch abgedichteten Komponenten (976) mit dem leitenden Bereich (104) und/oder einer Komponente außerhalb des Elektronikgehäuses elektrisch zu verbinden.

6. Verfahren nach Anspruch 5, wobei das Bereitstellen des Substrats umfasst:
Verbinden einer integrierten Schaltung, die die eine oder die mehreren aktiven Komponenten enthält, mit der Oberfläche eines Substrats.

7. Verfahren nach Anspruch 5, wobei das Bereitstellen des Substrats umfasst: Fertigen der einen oder der mehreren aktiven Komponenten direkt in der Oberfläche des Substrats.

8. Verfahren nach Anspruch 5, das ferner umfasst: elektrisches Verbinden des leitenden Bereichs mit einem zweiten Elektronikgehäuse.

9. Verfahren nach Anspruch 5, wobei das Bilden des leitenden Bereichs (104) umfasst: Ablagern einer metallischen dünnen Schicht auf der freiliegenden Oberfläche des Substrats und/oder der Abdeckung.

10. Verfahren nach Anspruch 5, wobei das Substrat mehrere Bereiche umfasst, wovon jeder eine oder mehrere aktive Komponenten enthält, und wobei das Verfahren ferner umfasst:
Verbinden einer Abdeckung (640), die mehrere Komponenten besitzt, mit dem Substrat (624) derart, dass alle der mehreren Bereiche des Substrats (624) gegenseitig hermetisch abgedichtet sind.

11. Verfahren nach Anspruch 10, das ferner umfasst:
Trennen der mehreren Bereiche voneinander derart, dass das Substrat in mehrere hermetisch abgedichtete und physikalisch getrennte Module unterteilt ist, wovon jedes einen leitenden Bereich auf einer äußeren Oberfläche hiervon besitzt.

12. Verfahren nach Anspruch 5, das ferner umfasst: Testen der Integrität der hermetischen Dichtheit zwischen dem Substrat (124) und der Abdeckung (140) durch Ausführen eines Lecktests mittels eines optischen Interferometers, um die Integrität der hermetischen Abdichtung zu testen.

13. Elektronikgehäuse nach Anspruch 1, wobei die eine oder die mehreren aktiven Komponenten eine integrierte Schaltung umfassen, die mit dem Substrat und/oder der Abdeckung verbunden sind.

14. Elektronikgehäuse nach Anspruch 1, wobei eine oder mehrere aktive Komponenten direkt in der Oberfläche des Substrats und/oder der Abdeckung gebildet sind.

15. Elektronikgehäuse nach Anspruch 1, wobei ein leitender Pfad (994) für einen Rahmen vorgesehen ist, indem er zwischen dem Rahmen und dem Substrat (986) und/oder einem zweiten Rahmen (994) und/oder der Abdeckung (984) vorgesehen ist.

16. Elektronikgehäuse nach Anspruch 1, wobei ein leitender Pfad für einen Rahmen (994) als eine Öffnung durch diesen Rahmen vorgesehen ist.

## Revendications

1. Boîtier électronique (102) pour un dispositif médical implantable actif (DMIA), comprenant :
un substrat planaire biocompatible, électriquement non conducteur et imperméable au fluide(986), utilisable pour la fabrication de semiconducteur ;
un ou plusieurs composants actifs (976) ;
un premier encadrement biocompatible, électriquement non conducteur et imperméable au fluide (974A), fixé à une surface du substrat (986), de telle sorte que l'encadrement (974A) entoure le périmètre de l'un ou de plusieurs des composants actifs (976) ;
un ou plusieurs seconds encadrements biocompatibles, électriquement non conducteurs, imperméables au fluide (974), fixés à une surface du premier encadrement (974A) ou à l'autre second encadrement (974B) ;
un couvercle biocompatible et imperméable au fluide (984), lié à une surface d'un second encadrement (974C) pour fermer hermétiquement l'un ou plusieurs des composants actifs (976) entre le substrat (986) et le couvercle (984) ;
une région conductrice (104) formée sur au moins une d'une surface exposée du substrat (986) et le couvercle (984) électriquement relié à au moins un de l'un ou de plusieurs des composants actifs fermés hermétiquement (976) ; et
au moins un chemin conducteur (994) est prévu pour chaque encadrement (974) pour relier électriquement au moins un de l'un ou de plusieurs des composants actifs fermés hermétiquement (976) avec au moins une de la région conductrice (104) et un composant externe au boîtier électronique.

2. Boîtier électronique selon la revendication 1, dans lequel la région conductrice (104) est configurée pour s'intégrer avec le tissu d'un receveur du DMIA, et dans lequel au moins un de l'un ou de plusieurs des composants actifs comprennent un stimulateur configuré pour générer des signaux de stimulation électrique délivrés au tissu par la région conductrice (104).

3. Boîtier électronique selon la revendication 1, dans lequel la région conductrice (104) est configurée pour intégrer avec le tissu d'un receveur du DMIA, et dans lequel la région conductrice est configurée pour détecter une impulsion nerveuse générée par le tissu, et transmettre un signal électrique représentant l'impulsion nerveuse à au moins un composant actif relié électriquement à celui-ci.

4. Boîtier électronique selon la revendication 1, comprenant en outre une seconde région conductrice configurée pour être reliée électriquement à un plot de connexion (1280) d'un second boîtier électronique (1202A).

5. Procédé de fabrication d'un boîtier électronique (102) pour un dispositif médical implantable actif (DMIA), comprenant :
la fourniture (302) d'un substrat planaire biocompatible, électriquement non conducteur, imperméable au fluide (986), ayant un ou plusieurs composants actifs (976) ;
la liaison (308) d'un premier encadrement biocompatible, électriquement non conducteur, imperméable au fluide (974A) à la surface du substrat (986), de telle sorte que l'encadrement (974A) entoure le périmètre de l'un ou de plusieurs des composants actifs (976) ;
la liaison d'un ou plusieurs seconds encadrement biocompatible, électriquement non conducteur, imperméable au fluide (974) à une surface du premier encadrement (974A) ou d'un autre second encadrement (974B) ;
la liaison (310) d'un couvercle biocompatible et imperméable au fluide (984) à une surface d'un second encadrement (974C) pour fermer hermétiquement l'un ou plusieurs des composants actifs (976) entre le substrat (986) et le couvercle (984) ;
la formation (310) d'une région conductrice (104) sur au moins une surface exposée du substrat (986) et le couvercle (984), la région conductrice étant reliée électriquement à au moins un de l'un ou de plusieurs des composants actifs fermés hermétiquement (976) ; et
la formation d'au moins un chemin conducteur (994) pour chaque encadrement (974) pour relier électriquement au moins un de l'un ou de plusieurs composants actifs fermés hermétiquement (976) avec au moins une de la région conductrice (104) et un composant externe au boîtier électronique.

6. Procédé selon la revendication 5, dans lequel la fourniture du substrat comprend :
la liaison d'un circuit intégré comprenant le un ou plusieurs composants actifs à la surface d'un substrat.

7. Procédé selon la revendication 5, dans lequel la fourniture du substrat comprend :
la fabrication de l'un ou plusieurs composants actifs directement sur la surface du substrat.

8. Procédé selon la revendication 5, comprenant en outre : la liaison électrique de la région conductrice à un second boîtier électronique.

9. Procédé selon la revendication 5, dans lequel la région conductrice (104) comprend : le dépôt d'un film mince métallique sur la au moins une d'une surface exposée du substrat et du couvercle.

10. Procédé selon la revendication 5, dans lequel le substrat comprend une pluralité de régions comprenant chacune ou plusieurs composants actifs, et dans lequel le procédé comprend en outre :
la liaison d'un couvercle (640) ayant une pluralité de composants au substrat (624), de telle sorte que chacune de la pluralité de régions du substrat (624) est protégée hermétiquement d'une autre.

11. Procédé selon la revendication 10, comprenant en outre :
la séparation de la pluralité de régions l'une de l'autre, de telle sorte que le substrat soit divisé en une pluralité de modules séparés physiquement et fermés hermétiquement, chacun ayant une région conductrice sur une de ses surfaces extérieures.

12. Procédé selon la revendication 5, comprenant en outre : le contrôle de l'intégrité de la fermeture hermétique entre le substrat (124) et le couvercle (140), en procédant à un test d'étanchéité par interféromètre optique, pour vérifier l'intégrité de la fermeture hermétique.

13. Boîtier électronique selon la revendication 1, dans lequel un ou plusieurs composants actifs comprennent un circuit intégré lié à au moins un du substrat et du couvercle.

14. Boîtier électronique selon la revendication 1, dans lequel un ou plusieurs composants actifs sont formés directement à la surface d'au moins un du substrat et du couvercle.

15. Boîtier électronique selon la revendication 1, dans lequel un chemin conducteur (994) est prévu pour un encadrement, en étant placé entre cet encadrement et un du substrat (986), d'un autre second encadrement (994) ou d'un couvercle (984).

16. Boîtier électronique selon la revendication 1, dans lequel un chemin conducteur est prévu pour un encadrement (994) en tant qu'ouverture à travers cet encadrement.
